Europäisches Patentamt

European Patent Office

Office européen des brevets

(19)

(11) Publication number : **0 581 581 A2**

# EUROPEAN PATENT APPLICATION

(12)

(21) Application number : 93305950.3

(22) Date of filing : 28.07.93

(51) Int. Cl.$^5$ : **A61K 9/70,** A61L 15/24, A61M 35/00

(30) Priority : 29.07.92 US 921819

(43) Date of publication of application :
02.02.94 Bulletin 94/05

(84) Designated Contracting States :
AT CH DE GB IT LI SE

(71) Applicant : JOHNSON & JOHNSON
CONSUMER PRODUCTS, INC.
Grandview Road
Skillman, New Jersey 08558 (US)

(72) Inventor : Wang, Jonas C. T.
23 Ellsworth Dr.
Robbinsville, NJ 08691 (US)
Inventor : Liu, Jue-Chen
29 Van Bolton Rd.
Neshanic, NJ 08853 (US)
Inventor : Hudson, Marilyn
109 Shady Lane
Randolph, NJ 07869 (US)

(74) Representative : Fisher, Adrian John et al
CARPMAELS & RANSFORD 43 Bloomsbury
Square
London WC1A 2RA (GB)

(54) Bioadhesive treatment compositions and methods of use.

(57) A composition comprising a dispersion of a bioadhesive containing polyvinyl pyrrolidone and a carboxy-functional polymer, and water is disclosed. The pH of the composition is controlled, preferably to about 6.0 or less, so that the bioadhesive is in a water-insoluble form. The bioadhesive is capable of binding water and other suitable ingredients, such as cosmetic or medicinal agents, to a mucosal membrane or the skin. If it is desirable to remove the composition, it may be washed away with ordinary tap water. Upon contact with tap water, the bioadhesive becomes water-soluble and loses its adhesive characteristics. A method of adhering the composition to the surface of a mucosal membrane or the skin is also disclosed.

EP 0 581 581 A2

## Field of the Invention

The present invention relates to bioadhesive treatment compositions which adhere to the skin and mucosal membranes, and to methods utilizing these compositions.

## Background of the Invention

Bioadhesives are generally known to be materials that are capable of being bound to a biological membrane, and retained on that membrane for an extended period of time. Bioadhesive compositions often include a therapeutic agent, which is released in a controlled fashion to provide a longlasting effect.

U.S. Patent No. 4,292,299 to the Suzuki, et al., describes controlled release preparations in the form of tablets or granules, which contain a bioadhesive layer and a non-adhesive layer containing a medicament. When the preparation is exposed to the wet mucosal surface, the bioadhesive layer becomes adhered, but the non-adhesive layer begins to disintegrate thereby releasing the medicament. Polymers suitable for use in these preparations include acrylic acid homopolymers and copolymers, hydrophilic vinyl polymers, hydrophilic cellulose derivatives, and polysaccharides. Mixtures of these polymers, such as polyvinyl pyrrolidone and carboxymethyl cellulose, are also described as being useful as the adhesive layer.

Bioadhesive compositions suitable for use as adhesives for oral mucosal membrane plasters are discussed in Japanese Laid Open Patent Application No. 63-189484. The adhesive compositions comprise polyvinyl pyrrolidone, carboxymethyl cellulose or a salt thereof, and a water softening agent, such as glycerin, diglycerin, triglycerin, sorbitol and multitol. The weight ratio of the polyvinyl pyrrolidone to carboxymethyl cellulose is within the range of 95:5 to 60:40, and the content of the water-retaining softener with respect to 100 parts of the aforementioned polymers is within the range of 20 to 400 parts by weight. The components of the composition are first dissolved individually in water, alcohol, etc., and the resulting solutions are then mixed in the specified ratios to prepare a solution of the adhesive composition. This solution is cast and dried, and used as the adhesive layer in the oral mucous membrane plaster. The plaster film can be used to protect localized areas of the oral mucosal membrane by applying the plaster to the affected area. Alternatively, the dried plaster may be impregnated with an active drug and used in preparations which provide a systemic therapeutic effect by absorption of the drug through the oral mucosal membrane.

U.S. Patent No. 4,615,697 to J. R. Robinson relates to a controlled release treatment composition which, when in the presence of sufficient water to swell the bioadhesive, will adhere to skin or to a mucosal membrane. The bioadhesive component of a composition contains a water-swellable, but water-insoluble, fibrous, crosslinked carboxyfunctional polymer, such as polycarbophil. The treating agents useful in the composition are selected from medicinal agents and cosmetic agents known in the art.

Bioadhesive compositions containing polycarbophil are commercially available. For example, Warner-Lambert Co. markets a vaginal moisturization product, REPLENS®, containing polycarbophil which becomes bound to the vaginal cell surface. Moisture, within the polycarbophil polymer matrix, is delivered to the vaginal cell surface in a sustained release fashion. Warner-Lambert reports that the REPLENS® product is capable of hydrating the underlining vaginal tissue for days, thus eliminating the need for daily application of the product by women who suffer from vaginal dryness.

Since the REPLENS® product contains a water-insoluble bioadhesive polymer and is formulated as an oil-in-water emulsion, it is difficult to remove the product from mucosal tissue with ordinary tap water. While this is a desirable property for longlasting moisturization of vaginal tissue, it is an undesirable property when one wishes to remove the product from the vagina.

It would be desirable to have a product in which the bioadhesive properties can be selectively reversed so as to facilitate easy removal. Thus, a need has arisen for a composition which will bioadhesively attach to a mucosal membrane, but can be removed by natural physiological cell turn over or with tap water.

## SUMMARY OF THE INVENTION

The present invention relates to a composition employing a bioadhesive containing polyvinyl pyrrolidone and a carboxy-functional polymer, which is dispersed in a pharmaceutically acceptable vehicle. The vehicle is preferably water, in an amount sufficient to form a liquid suspension, preferably a gel. The pH of the composition is controlled, preferably to about 6.0 or less, so that the bioadhesive is in a water-insoluble form. When in the water-insoluble form, the bioadhesive is capable of binding water and other suitable ingredients to a mucosal membrane or the skin. If it is desirable to remove the composition, it may be washed away with ordinary tap water, generally having a pH within the range of about 6.5-8.0. Upon contact with the tap water, the bioadhesive dissociates, becomes water-soluble, and loses its adhesive characteristics.

The composition of the present invention may also include a treating agent, such as a cosmetic or medicinal agent. When employed in the composition, the treating agent and water become bioadhesively bound to the mucosal membrane or skin. When the treating agent is a personal lubricant, the composition may be used as a sexual lubricant and/or a moisturizer for alleviating the symptoms of vaginal dryness.

The present invention also includes a method of adhering the composition to the surface of a mucosal membrane or the skin by contacting the membrane or skin with a bioadhesive containing polyvinyl pyrrolidone and a carboxy-functional polymer, which is dispersed in a pharmaceutically acceptable vehicle.

BRIEF DESCRIPTION OF THE DRAWINGS

Figure 1 is a graph illustrating the in vitro bioadhesive force of a personal lubricant composition of the present invention and several comparative personal lubricant compositions.

Figure 2 is a graph illustrating the relative amounts of the personal lubricant of the present invention and a comparative composition remaining in a test subject's vagina over an extended period of time.

Figure 3 is a graph illustrating the percent weight change of the personal lubricant of the present invention over time when immersed in buffered solutions having pHs ranging from 3-8.

Figure 4 is a graph illustrating the percent weight change of a comparative personal lubricant over time when immersed in buffered solutions having pHs of 4 and 8.

Figure 5 is a graph illustrating the weight percent of dry solids of the personal lubricant of the present invention remaining after 24 hours in a series of buffered solutions having pHs ranging from 3-8.

Figure 6 is a graph illustrating the weight percent of dry solids of the personal lubricant of the present invention and a comparative lubricant remaining after 24 hours in buffered solutions having pHs of 4 and 8.

Figure 7 is a graph illustrating the amount of water retained by a personal lubricant of the present invention after 24 hours in a series of buffered solutions having pHs ranging from 3-8.

DETAILED DESCRIPTION OF THE PREFERRED EMBODIMENTS

The composition of the present invention includes a bioadhesive containing polyvinyl pyrrolidone and a carboxy-functional polymer, dispersed in a pharmaceutically acceptable vehicle. The pH of the composition is controlled, preferably to about 6.0 or less, so that the bioadhesive is present in an insoluble form. While in the insoluble form in the dispersion, the solid bioadhesive may swell in the pharmaceutically acceptable vehicle, especially when the vehicle is water. If the bioadhesive is subjected to a higher pH, it dissociates, becomes soluble in the vehicle and loses its adhesive characteristics.

The pharmaceutically acceptable vehicle is the bulk substance through which the bioadhesive is distributed to form a dispersion. This vehicle must also be capable of being used in or on humans or other mammals without causing any ill effects, such as toxicity or severe irritation to the skin or mucosal tissue. Pharmaceutically acceptable vehicles suitable for use in the present invention include water, alcohols, such as ethanol and propylene glycol, and mixtures thereof. The dispersion may take the form of a suspension, cream, suppository, lotion, mousse, aerosol spray or ointment. The composition is preferably a liquid suspension, such as a gel, employing water as the vehicle.

The composition contains a sufficient amount of the vehicle to form any of the aforementioned dispersions. The composition generally comprises from about 30 to about 95, preferably from about 50 to about 85, weight percent of the vehicle, such as water.

The bioadhesive component of the composition contains polyvinyl pyrrolidone and a carboxy-functional polymer. The polyvinyl pyrrolidone has a molecular weight of 1,200-1,200,000 and a viscosity of 1-30 centipoise in a 1% solution at room temperature (22° ± 1° C). Carboxy-functional polymers suitable for use in the bioadhesive include polyacrylic acid, carboxymethyl cellulose, and polymethylacrylic acid. These carboxy-functional polymers have a molecular weight of 90,000-700,000 and a viscosity of 50-600 centipoise in a 1% solution at room temperature.

The composition generally contains from about 0.1 to about 10, preferably from about 0.5 to about 2.0, by weight of the bioadhesive. The weight ratio of polyvinyl pyrrolidone to carboxy functional polymer is within the range of about 0.01:1 to about 5:1. When carboxymethyl cellulose is employed as the carboxy-functional polymer, the weight ratio of polyvinyl pyrrolidone to carboxymethyl cellulose is within the range of about 0.1:1 to about 4:1, preferably about 0.5:1 to about 2:1 and most preferably about 1:1.

The pH of the composition is about 6.0 or less. When the composition is to be used on the mucosal membrane of a woman's vagina, the pH is preferably within the range of about 3.0 to about 5.0 to match the natural pH of the vagina. The composition pH is affected by the concentration of the carboxy-functional polymers, but is controlled through the addition of suitable buffers such as citric acid, acetic acid, sodium acetate, tartaric

3

acid, lactic acid and glucono-delta-lactone.

While wishing not to be bound by any theory, it is believed that, through hydrogen bonding, the polyvinyl pyrrolidone and carboxy-functional polymer form a water insoluble complex This complex is water-insoluble when the composition pH is about 6.0 or less. However, when the composition becomes more alkaline, the complex dissociates to a water soluble form.

It should be noted that the concentrations and ratios of the polyvinyl pyrrolidone and carboxy-functional polymers described herein refer to both the complexed and uncomplexed forms of these polymers.

When the composition of the present invention is a personal lubricant in the form of a gel, it generally contains:

about 0.5 to about 2.0 percent by weight hydroxyethyl cellulose;

about 0.5 to about 2.5 percent by weight percent of the bioadhesive containing polyvinyl pyrrolidone and carboxymethyl cellulose in a weight ratio (polyvinyl pyrrolidone:carboxymethyl cellulose) of from about 0.1:1 to about 4:1, preferably about 0.5:1 to about 2:1 and most preferably about 1:1;

about 10 to about 40 percent by weight glycerine or propylene glycol; and

about 50 to about 85 percent by weight water, and has a pH of about 3.0 to about 5.0.

Although the composition containing simply the bioadhesive and water is useful for hydrating a mucosal membrane or skin tissue, the compositions of the present invention may also contain a treating agent. The treating agent is delivered to the skin or mucosal membrane in a sustained release fashion. As used herein, mucosal membrane is meant to include the membrane walls of oral, buccal, ocular, nasal, vaginal and rectal cavities.

The treating agents suitable for use in the present invention include any cosmetic or medicinal agent. These agents include personal lubricants, antiviral agents, antifungal agents, antibacterial agents, contraceptives, deodorants, fragrances, anesthetics, ophthalmic products, analgesics, antipurities, antihistamines, steroids, skin protectants, sunscreens, counterirritants, anticarries agents, anti-inflammatory, steroid replacements, proteins/peptides, and other drugs. One or more of these treatment agents may be employed in the composition of the present invention to provide the desired effect.

Suitable personal lubricants include hydroxyethyl cellulose, glycerine, mineral oil, petrolatum, silicones, lanolin, lanolin oil, polyethylene glycol and triglycerides.

Antiviral agents suitable for use in the present invention include nonoxynol-9, octoxynol-9 and menfegol, which are also known to have spermicidal activity, and zinc salts, such as zinc gluconate and zinc acetate.

The bioadhesive of the present invention may also be used with antifungal agents such as miconazole, ketoconazole, tolnaftate, undecylenic acid and sodium propionate.

Contraceptives suitable for use in the present invention include nonoxynol-9, octoxynol-9, menfegol dodecaethylene glycol monolaurate, Laureth IOS, methoxypolyoxyethylene glycol laurate-550.

The composition may also have antibacterial properties through the addition of antibacterial agents such as benzethonium chloride, benzalkonium chloride, methylbenzethonium chloride, povidone iodine and chlorohexidine gluconate.

Deodorants and fragrances useful in the present invention include sodium bicarbonate, aluminum chloride, aluminum chlorohydrates, aluminum zirconium chlorohydrates, buffered aluminum sulfate, trioclosan and trichlorocarbanilide.

The treating agents are present in the composition in any therapeutic or cosmetically effective amount. Generally, they are present in an amount ranging from about 0.004 to about 25, preferably from about 0.1 to about 15, by weight of the composition.

In addition to the bioadhesive, treating agent and water, the composition may also contain pharmaceutically or cosmetically acceptable additives. These additives include stabilizers, preservatives, excipients, binders, vehicles, chelating agents, antioxidants, coloring agents, flavors, odor controlling agents and the like.

The composition of this invention may be administered by virtually any means to provide the desired contact between the skin or mucosal membrane and the composition. If the composition is to be applied to the skin, it is simply rubbed over the area to be treated. The composition is applied to the buccal, ocular, nasal, vaginal or rectal mucosal membranes with the hand, forceps or other suitable instruments. Irrespective of the method of application, the composition is left in contact with the skin or mucosal membrane for a sufficient time for the treating agent to be released over a controlled period.

Example 1

A personal lubricant gel of the present invention was prepared having the formulation shown in Table 1.

TABLE 1

| Ingredient | Formulation 1 (% by Weight) |
|---|---|
| Carboxymethyl cellulose | 1.0 |
| Polyvinyl pyrrolidone | 1.0 |
| Hydroxyethyl cellulose | 1.5 |
| Glycerine | 17.0 |
| Methylparaben | 0.15 |
| Sorbic acid | 0.10 |
| Citric acid | 0.35 |
| Water | 78.90 |

This gel was prepared by premixing at 85°C the preservatives (methylparaben and sorbic acid) and glycerine. The premix was then combined with the remaining components of the composition and then mixed to form a hydrated gel at 31°C.

The resulting gel was clear and had a pH of 3.8-4.2.

Example 2

A study was conducted to measure the in-vitro bioadhesive force of Formulation 1 from Example 1 and three comparative formulations. Comparative A was a non-bioadhesive lubricating jelly which was prepared using the preparation procedure described above for Example 1. Comparative B was REPLENS® vaginal moisturizer. The compositions of Comparatives A and B are shown in Table 2. Comparative C was KV Moisturizer available from KV Pharmaceutical Co. KV Moisturizer is a vaginal moisturizing/lubricating product containing a bioadhesive, and is formulated as a water-in-oil emulsion.

## TABLE 2

| Ingredients | Comparative A | Comparative B |
| --- | --- | --- |
| | (% by Weight) | |
| Bioadhesive Polymer(s) | ... | Polycarbophil Carbopol |
| Lubricity Agent | Hydroxyethyl Cellulose (2.1) | ... |
| Cosolvent/ Lubricity | Glycerine (17) | Glycerine (11) |
| Emulsifier | ... | H-Palm Oil Glyceride |
| Preservatives | Methyl-paraben (0.2) Chlorhexidine Gluconate (0.05) | Methyl paraben (0.175) Sorbic Acid (0.032) |
| Oil | ... | Mineral Oil |
| Buffers | Gluconodelta Lactone (0.5) | ... |
| Water | (q.s. to 100) | (approx. 80) |
| Formulation Type | Gel | Lotion (oil-in-water emulsion) |

... = not present

The in-vitro bioadhesive force was measured on a Chatillon Digital Force Gauge Model DFGHS 2 available from Chatillon Instruments. Two pieces of chamois, each having a diameter of 1.3 cm and an area of 1.3 cm$^2$, were used as the membranes on the force gauge. One membrane was attached to the stationary steel platen with epoxy glue. The other membrane was attached with a waterproof adhesive to an acrylic plate, which could be raised or lowered at a variable rate. The membranes were equilibrated with a citrate-phosphate buffer having an ionic strength of 0.1M and a pH of 4.

The membranes were equilibrated for one minute at 37°C. The test formulation (0.3 ml sample size) was then applied to the membrane mounted on the steel platen using a syringe. The buffer solution used to equilibrate the membrane was also used as the control solution. The moveable acrylic plate containing the other chamois membrane was moved vertically until it contacted the stationary plate and a compressive force of one pound was obtained. The moveable plate and the stationary platen were maintained in this condition for one minute. Then, the moveable plate was lowered at a slow, controlled rate for 30 seconds and bioadhesive force of the test formulation was measured.

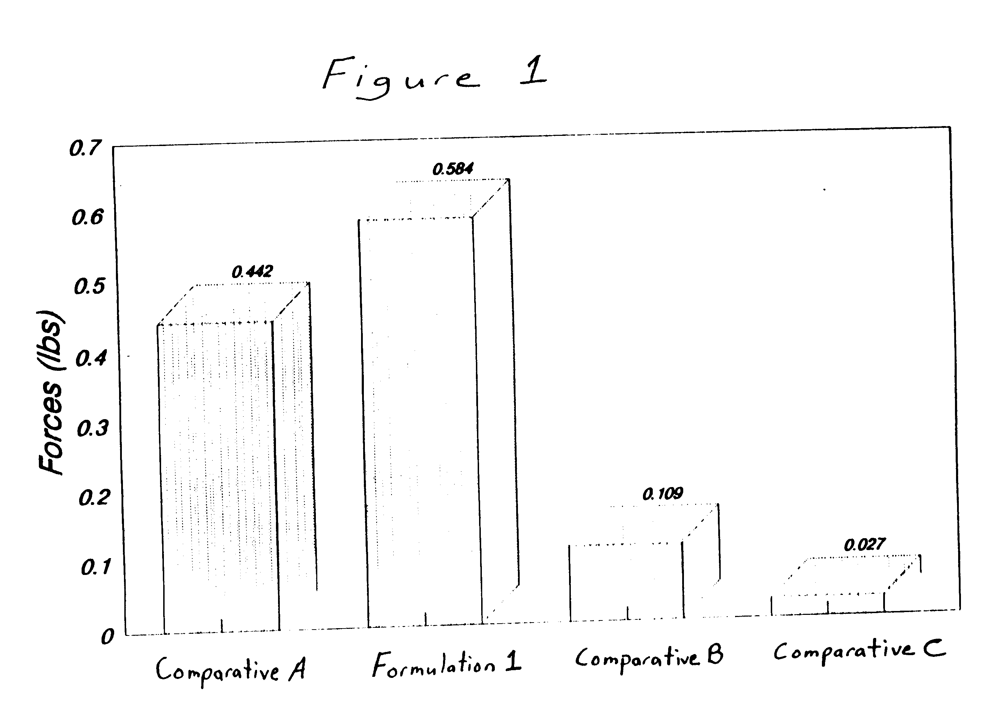

FIG. 1 reports the bioadhesive force of each of the tested formulations. The results presented in this figure show that the personal lubricant of the present invention has a greater bioadhesive force than the comparative formulations, especially Comparatives B and C containing the bioadhesive polymers.

Example 3

A study was conducted to measure the amount of the personal lubricant which was retained within a human subject's vagina over an extended period of time. In this study, a personal lubricant composition of the present invention, identified as Formulation 2 in Table 3, was compared with Comparative A from Table 2. Formulation 2 was prepared in accordance with the procedure used for Example 1. Both Comparative A and Formulation 2 were gels. Two clinical studies, using the same protocol, were conducted in which adult female subjects were randomly assigned to use either the Formulation 2 or Comparative A. A total of 41 adult females completed this study, in which 20 used Formulation 2 and 21 used Comparative A.

TABLE 3

| Ingredient | Formulation 2 (% by Weight) |
|---|---|
| Carboxymethyl cellulose | 1.0 |
| Polyvinyl pyrrolidone | 0.9 |
| Hydroxyethyl cellulose | 1.5 |
| Glycerine | 17.0 |
| Methyl paraben | 0.2 |
| Sorbic acid | 0.05 |
| Citric acid | 0.35 |
| Water | 79.0 |

Prior to introduction of the lubricant product, blank samples were taken from all subjects. The formulations were then vaginally introduced into the subjects. Samples were then taken at 1, 5, 10 and 24 hour intervals after introduction of the products. The samples were taken with cotton swabs which were weighed before and after swabbing to determine the sample weights. The sample swabs were then placed in screw-cap vials containing 2 ml. of ethanol:water (30:70) until analyzed.

Both Formulation 2 and Comparative A contain 17% by weight glycerine. The swab samples were analyzed for glycerine using gas chromatography (GC). In the GC analysis, a small interfering ghost peak was observed when the methanol:water blank was injected. The standard and sample areas were corrected for the interfering peak. The set of five samples (blank, 1 hour, 5 hour, 10 hour and 24 hour) obtained from each subject was then analyzed in duplicate. Before analyzing a sample set, three injections of the standard were made. Blank solvent injections were made before and after the injection of the standards. The average area observed in the blank solvents was calculated, and the corresponding standards and the sample set areas were corrected by subtracting the average blank areas. Using this procedure, the amount of glycerine present in the examples was calculated using the following equation:

$$\% \text{ glycerine} = \frac{\text{sample area} \times 100 \times (2000 + \text{sample weight})}{\text{sample weight} \times \text{response factor for standard}}$$

where:

Response factor of standard is the glycerine peak area in the standard calculated for 1 mg/ml concentration;

Sample area is a glycerine peak area in the sample; and

Sample weight is in milligrams.

For the purposes of this test, it was assumed that the total volume of the sample solution is the volume of the methanol-water added (2 ml) and the weight of the sample.

From the percent glycerine, the percent lubricant resin in the samples was calculated as follows:

$$\% \text{ lubricant} = \frac{\% \text{ glycerine} \times 100}{17}$$

The results were tabulated and the resulting statistical analysis is shown in Table 4.

TABLE 4

Weight Percentage of Formulation in Vaginal Swabs

| Hour | Formulation | Median | Significance | Mean | Std. Deviation |
|---|---|---|---|---|---|
| 1 | Comp. A | 34.5 | ns | 33.5 | 20.4 |
|   | Form. 2 | 38.6 | ns | 38.4 | 21.9 |
| 5 | Comp. A | 0.4 |  | 13.9 | 30.2 |
|   | Form. 2 | 15.8 | p<.06 | 30.0 | 46.8 |
| 10 | Comp. A | 0.1 |  | 5.0 | 8.1 |
|   | Form. 2 | 10.0 | p<.05 | 12.0 | 11.8 |
| 24 | Comp. A | 0.0 |  | 0.8 | 1.6 |
|   | Form. 2 | 2.0 | p<.02 | 3.4 | 4.5 |

ns = not significant

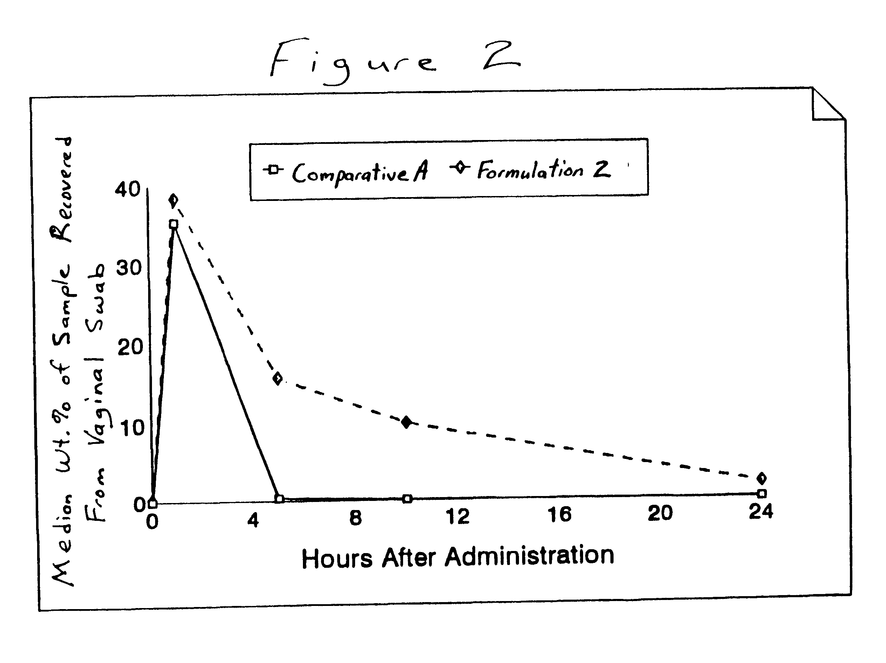

These results shown that after one hour, there was no statistical difference in the amount of the two tested lubricants remaining in the test subject's vagina. However, after 10 and 24 hours, it was found that there was a significantly higher amount of Formulation 2. This enhanced recovery shows that the personal lubricant of the present invention has a significantly longer lasting effect than the formulation without the bioadhesive. FIG. 2 is a plot of the median weight percent of the formulation recovered from the vaginal swab vs. the number of hours after administration.

8

Example 4

A study was conducted to determine the effect of pH on the dissolution of personal lubricant of the present invention, Formulation 2 of Example 3, and a comparative lubricant composition, Comparative A from Table 2, in water. The experiment was conducted using a United States Pharmacopeia Dissolution Apparatus 1. Known quantities of each lubricant were placed in a basket and then immersed in a vessel filled with aqueous buffers of various pHs. The weight change of the sample over time was monitored and the residual, sample dried weight after dehydration was measured at the conclusion of the experiment.

Citrate-phosphate buffer solutions (ionic strength equal 0.1M) were prepared having pHs of 3, 4, 5, 6, 7 and 8. The dissolution flasks in the apparatus were each filled with separate buffer solutions and equilibrated at 37°C for one hour. Prior to charging with the sample, the empty basket was weighed. Then, one gram of each sample was loaded into a series of baskets which were then immersed in each of the buffered solutions. The rotation speed of the apparatus was set at 100 rpm and every basket was weighed at 0.5, 1, 2, 4, 6, and 24 hours. At the end of the experiment, the baskets with any remaining sample were dried in a desiccator overnight and the remaining solids were weighed. Each sample was run three times in each of the buffered solutions.

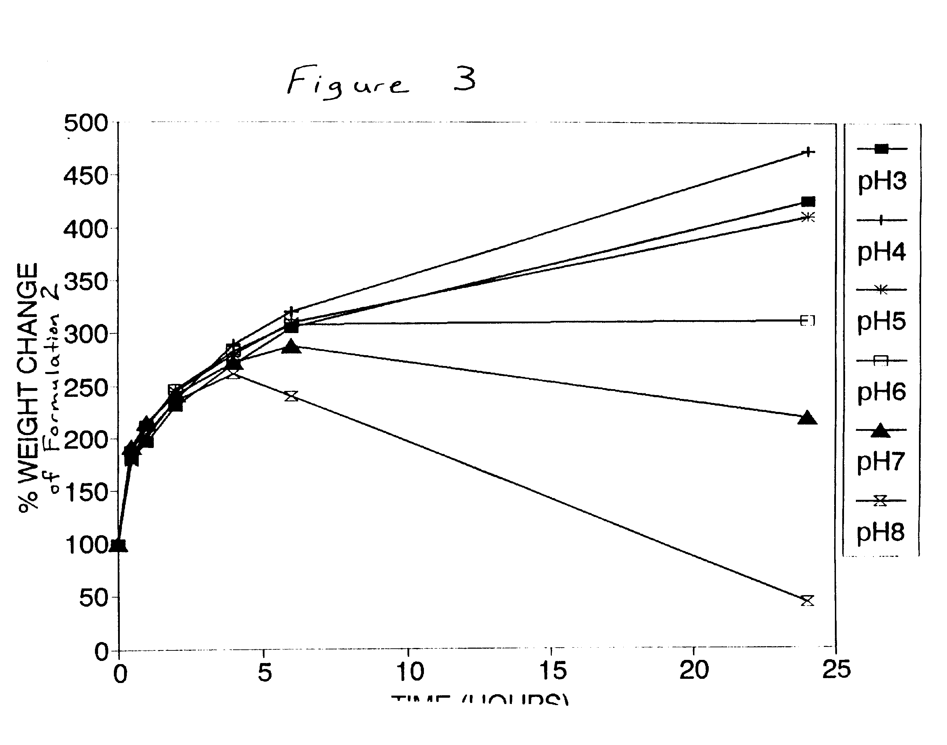

FIG. 3 is a graph showing the percent weight change of Formulation 2 over time. During the first four hours of the experiment, Formulation 2 swelled at similar rates at all pHs. However, after four hours, the dissolution phase started to dominate at pHs above 6 leading to a significant weight loss. At pHs of 6.0 or less, Formulation 2 after 4 hours generally continued to increase in weight, indicating further swelling of the bioadhesive.

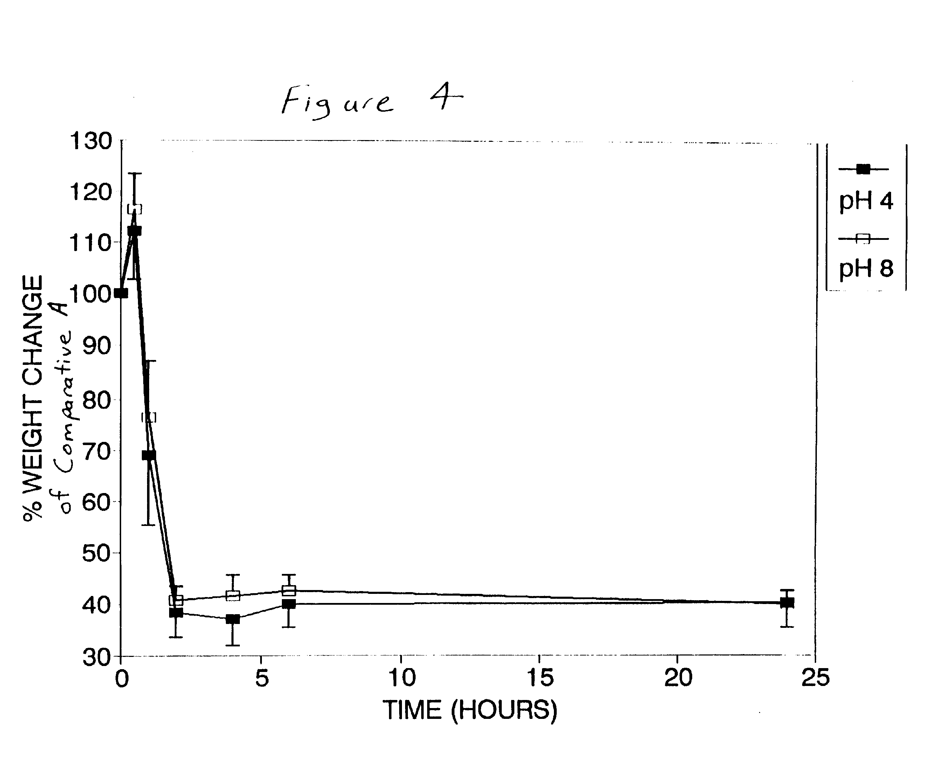

FIG. 4 shows the percent weight change for Comparative A vs. time. As can be seen, Comparative A experience significant weight loss almost immediately, indicating that the personal lubricant dissolved quickly, irrespective of the buffered solution.

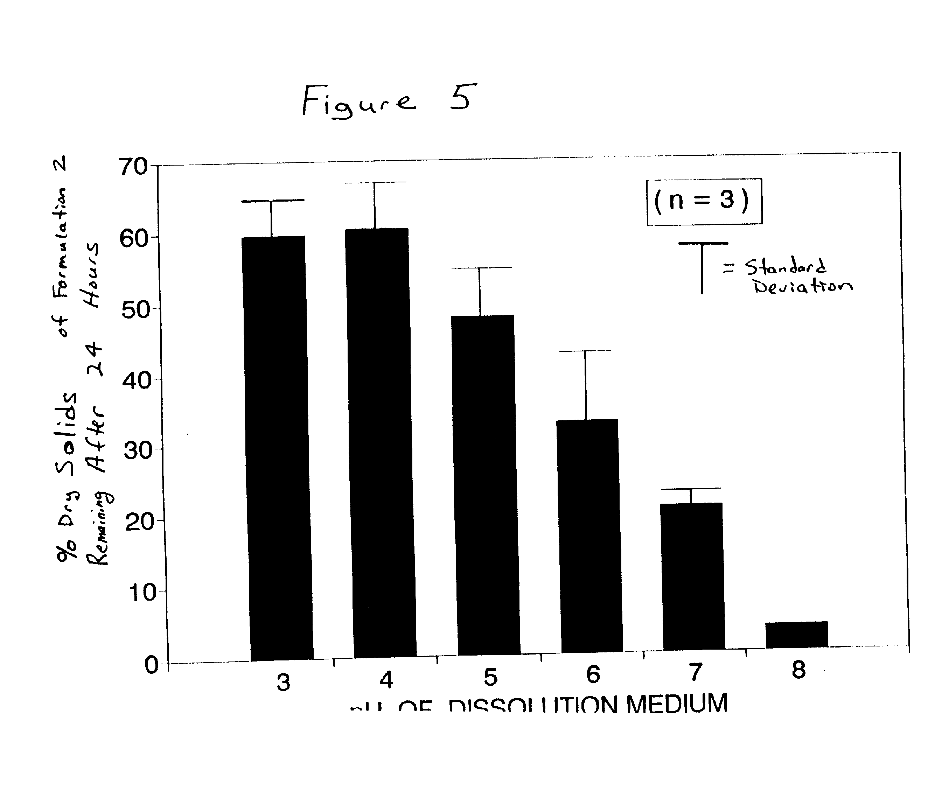

FIG. 5 shows the weight percentage of dry solids of Formulation 2 remaining after 24 hours. This percentage is based on the dry solids used to makeup the formulation. At pHs of 3-5, about 50% or more of the solids remained undissolved. In contrast, less than 5% of the solids remained at a pH of 8.

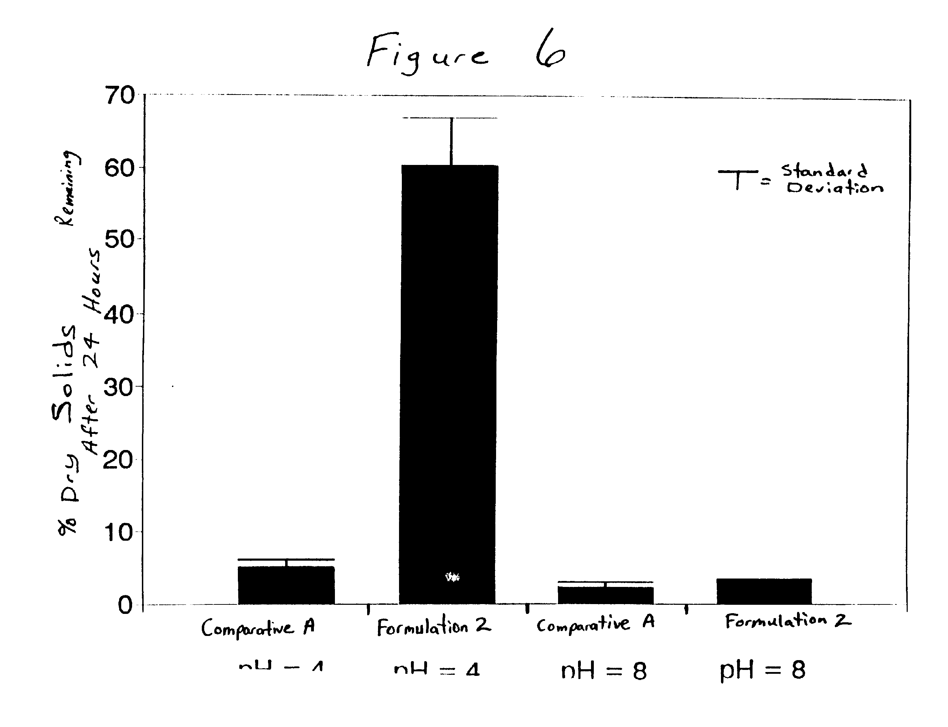

FIG. 6 is similar to FIG 5. and shows the weight percentage of dry solids of both Comparative A and Formulation 2 after 24 hours. Virtually all of Comparative A dissolved at pH of 4 and 8, whereas a substantial portion of the solids of Formulation 2 remained after 24 hours at a pH of 4.

Since the natural pH of a woman's healthy vagina is within the range of about 3-5, the personal lubricant of the present invention does not dissociate and retains its moisturizing and bioadhesive properties within the physiological pH range. However, if it is desirable to remove the composition from the mucosal tissue, these results show that the composition will dissociate with ordinary tap water having a pH in the range of about 6.5-8. The comparative personal lubricant without the bioadhesive was shown to dissociate irrespective of pH.

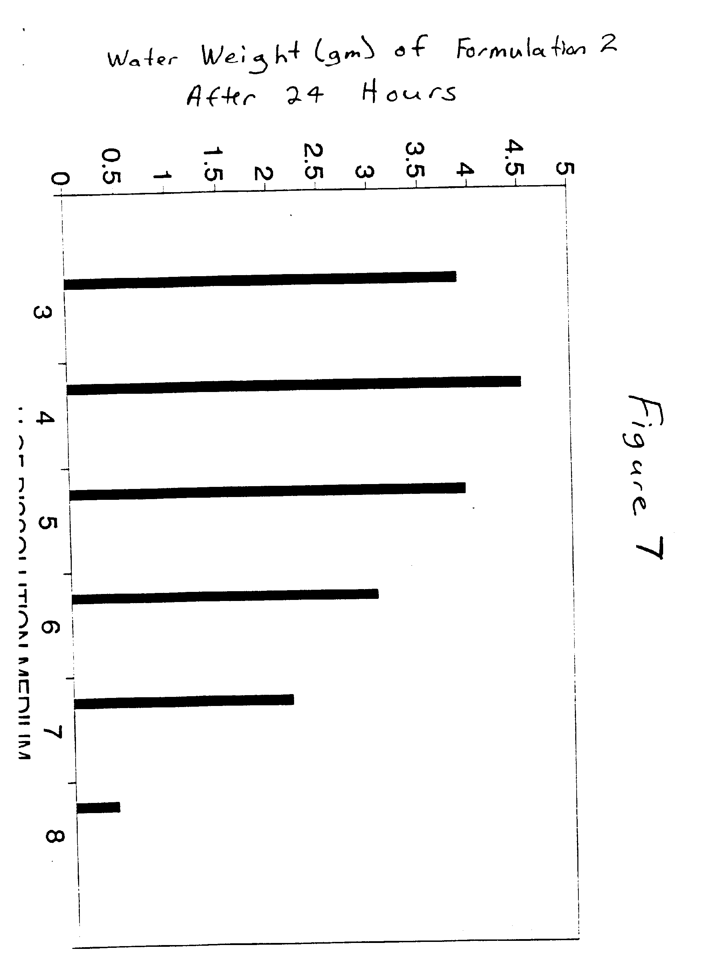

FIG. 7 shows the amount of water retained by Formulation 2 after 24 hours in the buffered solutions. A significantly higher amount of moisture was retained when the composition was maintained in an environment having a pH of 6 or less. This is an important property in a personal lubricant, since it indicates that the composition of the present invention provides maximum moisturization, i.e., water retention, at pHs associated with a healthy vagina.

Example 5

A test was conducted to measure the lubricity of the personal lubricant of the present invention, Formulation 2 of Example 3, and a comparative lubricant composition, Comparative A from Table 2. The coefficient of friction of the test sample was measured over time in an environment maintained at 30°C and 40% relative humidity.

The tests were conducted on a custom-made friction testing apparatus. The apparatus contained a probe which was moved over a fixed base. The probe was in the form of a spring biased wheel mounted about an axle. The axle was supported by a moveable platform which was disposed over the fixed base. The test sample was interposed between the wheel and the base, and the platform was moved parallel to the base while the wheel exerted a known amount of normal (or downward) force on the test sample and base. Based on the amount of wheel rotation, a computer was used to calculate the coefficient of friction of the test sample.

In order to more closely simulate the conditions under which a personal lubricant is used, a NU-GEL® hydrogel pad, available from Johnson & Johnson Medical, Inc, was secured to the base. The hydrogel pad was then covered with a natural membrane condom made from sheep caecum. A small block of styrofoam (85mm long, 25mm wide and 12mm thick) was secured to the wheel in such a way that the frictional forces sensed by the strofoam block were transmitted proportionally to the wheel, so that the coefficient of friction could be measured. The styrofoam was also covered with a sheep caecum membrane. The membranes were rinsed with water and kept moist by applying wet paper towels. The excess water was removed prior to testing by blotting with a paper towel.

The test sample (2 cc) was spread uniformly over the bottom of the block of styrofoam. The block/probe assembly was then placed on the base and the coefficient of friction was measured over a 15 minute period. Formulation 2 was tested once, whereas Comparative A was tested six times and the results were averaged.

During the first two minutes of the test, the coefficients of friction for Formulation 2 and Comparative A were 0.376 and 0.337, respectively. The coefficients of frictions for the last two minutes of the test (between minutes 13 and 15) for Formulation 2 and Comparative A were 0.455 and 0.628, respectively.

The increase in friction of the tested samples over time is attributed to sample drying. The coefficient of friction of Comparative A increased significantly because of this phenomenon. On the other hand, the lubricant of the present invention experienced a much lower increase in the coefficient of friction because the bioadhesive, initially swollen with water, hydrated the friction surfaces thereby providing a more lubricous environment.

The foregoing examples are intended to be illustrative of the present invention. Various changes and modifications can be made to the above-described embodiments without departing from the spirit and scope of the present invention.

## Claims

1. A composition, comprising:
   a bioadhesive dispersed in a pharmaceutically acceptable vehicle, said bioadhesive comprising polyvinyl pyrrolidone and a carboxy-functional polymer, and said composition having a pH sufficient to maintain said bioadhesive insoluble in said vehicle.

2. A composition according to claim 1, wherein said vehicle comprises water.

3. A composition according to claim 2, wherein the carboxy-functional polymer is selected from the group consisting of polyacrylic acid, carboxymethyl cellulose and polymethacrylic acid.

4. A composition according to claim 3, wherein water is the major constituent.

5. A composition according to claim 4, wherein the pH is about 6.0 or less.

6. A composition according to claim 5, in the form of a gel.

7. A treatment composition, comprising:
   a treating agent and
   a bioadhesive dispersed in water, said bioadhesive comprising polyvinyl pyrrolidone and a carboxy-functional polymer, said composition having a pH sufficient to maintain said bioadhesive in a water-insoluble form.

8. A personal lubricant composition, comprising:
   at least one personal lubricating agent and
   a bioadhesive dispersed in water, said bioadhesive comprising polyvinyl pyrrolidone and carboxymethyl cellulose, the weight ratio of polyvinyl pyrrolidone to carboxymethyl cellulose being within the range of about 0.01:1 to about 5:1, and said composition having a pH of about 6.0 or less.

9. A personal lubricant composition according to claim 8, comprising:
   about 0.5 to about 2.0 percent by weight hydroxyethyl cellulose;
   about 0.5 to about 2.5 percent by weight of said bioadhesive;
   about 10 to about 40 percent by weight glycerine or propylene glycol; and
   about 50 to about 85 percent by weight water, and said composition having pH of about 3.0 to about 5.0.

10. A method of adhering a composition to the surface of a mucosal membrane or skin, comprising:
    contacting the membrane or skin surface with a composition comprising a bioadhesive dispersed in a pharmaceutically acceptable vehicle, said bioadhesive comprising polyvinyl pyrrolidone and a carboxyfunctional polymer; and
    controlling the pH of said composition to maintain said bioadhesive insoluble in said vehicle, whereby the bioadhesive adheres to the membrane or skin surface.

Figure 1

Figure 2

Figure 3

% WEIGHT CHANGE
of Comparative A

Figure 4

% Dry Solids of Formulation 2
Remaining After 24 Hours

Figure 5

## Figure 6

% Dry Solids Remaining After 24 Hours

70
60
50
40
30
20
10
0

Comparative A — pH = 1
Formulation 2 — pH = 4
Comparative A — pH = 8
Formulation 2 — pH = 8

T = Standard Deviation

Water Weight (gm) of Formulation 2 After 24 Hours

Figure 7